(19) Europäisches Patentamt / European Patent Office / Office européen des brevets

(11) **EP 2 236 083 A1**

(12) **EUROPEAN PATENT APPLICATION**

(43) Date of publication:
**06.10.2010 Bulletin 2010/40**

(51) Int Cl.:
**A61B 5/151** $^{(2006.01)}$

(21) Application number: **10157750.0**

(22) Date of filing: **25.03.2010**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA ME RS**

(30) Priority: **31.03.2009 JP 2009084820**

(71) Applicant: **Sysmex Corporation
Kobe-shi, Hyogo 651-0073 (JP)**

(72) Inventor: **Hagino, Kei
Kobe-shi, Hyogo 651-0073 (JP)**

(74) Representative: **HOFFMANN EITLE
Patent- und Rechtsanwälte
Arabellastrasse 4
81925 München (DE)**

(54) **Puncture device and fine pore formation method**

(57)     A puncture device (1) includes a piston (40), wherein the needle is to be attached to a distal end of the piston; a drive spring (80) which has one end capable of contacting a proximal end of the piston, and moves the piston in a specific direction toward the skin; and a first contact section (52) including a contact surface capable of contacting other end of the drive spring. A length (C) between a supposed strike position and the contact surface of the first contact section is longer than a total of a natural length (A) of the drive spring and a length (B) between the proximal end of the piston and a tip end of the needle, wherein the supposed strike position is where the tip end of the needle is supposed to strike against the skin.

FIG. 23A     FIG. 23B     FIG. 23C

EP 2 236 083 A1

## Description

Field of the Invention

The present invention relates to a puncture device and a fine pore formation method.

Related Art

In order to measure a specific component such as glucose in a tissue fluid of a subject, for example, U.S. Patent Publication No. 2007/0233011 discloses a fine pore formation device which forms fine pores on a skin of a subject by puncturing the skin with a fine needle chip having many fine needles. According to this puncture device for forming fine pores, glucose is measured in such a way that, after a puncture action, a measurement device is mounted on a site of puncture and a tissue fluid is extracted from the skin.

In such measurement, it is preferable that extraction quantity and extraction speed of the tissue fluid are stable as much as possible for obtaining stable data regardless of person and puncture site. Therefore, it is preferable that a puncture degree by a fine needle, that is, a degree of fine pore formation is constant regardless of person and puncture site. A speed at which the fine needle strikes against the skin is considered as an influence on the degree of fine pore formation.

When the fine needle chip strikes against the subject's skin, the space between the eject position of the fine needle chip and a strike position of the fine needle chip against the skin differs in about several millimeters between the subject who has a much swelled skin at the puncture site and the subject who has a little swelled skin at the puncture site. Therefore, if the fine needle chip is accelerated or decelerated when the fine needle chip strikes against the skin, a speed at which the fine needle chip strikes against the skin varies depending on the strike position. In other words, a strike speed against the skin varies depending on degrees of the skin swell.

Further, because the skin to be selected as the puncture site is soft, when a pressure of the puncture device pressing against the skin becomes strong, the skin enclosed by a press member of the puncture device receives pressure from a surrounding area. Accordingly, the skin swells higher than a bottom surface (contact surface with the skin) of the press member. Further, even in the same front arms, there is difference in skin swell degree between a case where the press member is pressed against a site of relatively curved skin and a case where the press member is pressed against a site of flat skin. In other words, a skin swell degree varies depending on pressures against the skin by the puncture device and a pressed site. As a result, the strike position of the fine needle chip against the skin varies and eventually the strike speed against the skin varies.

SUMMARY OF THE INVENTION

The scope of the present invention is defined solely by the appended claims, and is not affected to any degree by the statements within this summary.

In accordance with a first aspect of the present invention, there is provided a puncture device for forming a fine pore on a skin of a subject by striking a needle against the skin, comprising: a piston, wherein the needle is to be attached to a distal end of the piston; a drive spring which has one end capable of contacting a proximal end of the piston, and moves the piston in a specific direction toward the skin; and a first contact section including a contact surface capable of contacting other end of the drive spring, wherein the one end of the drive spring and the proximal end of the piston are not fixed to each other, and/or the other end of the drive spring and the contact surface of the first contact section are not fixed to each other; and a length between a supposed strike position and the contact surface of the first contact section is longer than a total of a natural length of the drive spring and a length between the proximal end of the piston and a tip end of the needle, wherein the supposed strike position is where the tip end of the needle is supposed to strike against the skin.

According to a puncture device of the first aspect of the present invention, there is a specific interval in which a needle can move without receiving a bias force of a drive spring. The needle can be struck against a subject's skin in the specific interval. Therefore, it is possible to strike the needle against the subject's skin at a constant puncture speed without being influenced by variation of skin swell size. As a result, extraction quantity and extraction speed of the tissue fluid are stable. So, it is possible to obtain stable data regardless of person and puncture site.

It is preferable that the one end of the drive spring and the proximal end of the piston are not fixed to each other, and the other end of the drive spring and the contact surface of the first contact section are not fixed to each other.

It is preferable that the puncture device further comprise a repulsion spring for pushing back the piston in a direction opposite to the specific direction; and a second contact section including a contact surface capable of contacting one end of the repulsion spring, which is on a near side to the skin, wherein the piston comprises a repulsion spring reception section including a contact surface capable of contacting other end of the repulsion spring, which is on a distant side from the skin, the one end of the repulsion spring and the contact surface of the second contact section are not fixed to each other, and/or the other end of the repulsion spring and the contact surface of the repulsion spring reception section are not fixed to each other; and a length between the contact surface of the repulsion spring reception section and the tip end of the needle is longer than a total of a natural length of the repulsion spring and a length between the contact surface of the second contact section and the supposed strike position.

It is preferable that the one end of the repulsion spring and the contact surface of the second contact section are not fixed to each other, and the other end of the repulsion spring and the contact surface of the repulsion spring reception section are not fixed to each other.

The puncture device may further comprise a housing accommodating the piston, the drive spring, and the first contact section.

It is preferable that the housing comprises a skin contact surface for contacting the skin; and wherein the supposed strike position is arranged inside the housing by 0.2 to 0.8 mm from the skin contact surface of the housing.

It is preferable that the housing comprises a skin contact surface for contacting the skin; and wherein the supposed strike position is arranged inside the housing by 0.5 mm from the skin contact surface of the housing.

It is preferable that a puncture speed of the needle in the supposed strike position is 4 to 8 m/s.

It is preferable that the puncture speed of the needle in the supposed strike position is approximately 6 m/s.

The puncture device may further comprise a spring positioning section for positioning the drive spring between the proximal end of the piston and the contact surface of the first contact section.

It is preferable that the drive spring has tubular shape, the spring positioning section is a rod member extending from a center part of the contact surface of the first contact section through inner space of the drive spring toward the piston, and the piston comprises a passage for inserting the rod member inside the piston.

In accordance with a second aspect of the present invention, there is provided a fine pore formation method of forming a fine pore on a skin of a subject, comprising: a step of accelerating and moving a needle in a specific direction toward the skin by continuously transmitting an elastic energy which is stored in a drive spring to the needle; a step of releasing transmission of the elastic energy, thereafter the needle further moves to the specific direction; and a step of striking the needle against the skin.

The fine pore formation method may further comprise a step of storing the elastic energy in the drive spring by compressing the drive spring, wherein the needle is accelerated and moved by transmitting the stored elastic energy to the needle.

The fine pore formation method may further comprise a step of atttaching the needle to a specific member, wherein storing the elastic energy in the drive spring and atttaching the needle are simultaneously carried out.

It is preferable that a moving speed, when transmission of the elastic energy is released and the needle is moved further to the specific direction, is substantially constant.

It is preferable that the needle is decelerated in a case where the needle dose not strike against, the skin despite movement for a specific distance in the specific direction.

BRIEF DESCRIPTION OF DRAWINGS

FIG. 1 is a perspective view showing an overall configuration of an embodiment of a puncture device according to the present invention;
FIG. 2 is a perspective view showing an infernal configuration of the puncture device shown in FIG. 1;
FIG. 3 is an exploded perspective view of the puncture device shown in FIG. 1;
FIG. 4 is a front view showing an internal configuration of a rear cover of the puncture device shown in FIG. 1;
FIG. 5 is a perspective view showing an internal configuration of a front cover of the puncture device shown in FIG. 1;
FIG. 6 is a bottom view of a chip accommodation tool insertion member of the puncture device shown in FIG. 1;
FIG. 7 is a front view of an array chuck of the puncture device shown in FIG. 1;
FIG. 8 is a perspective view of a release button of the puncture device shown in FIG. 1;
FIG. 9 is a perspective view showing an overall configuration of a chip accommodation kit provided with a fine needle chip to be mounted on the puncture device shown in FIG. 1;
FIG. 10 is an exploded perspective view of the chip accommodation kit shown in FIG. 9;
FIG. 11 is a perspective view of the fine needle chip of the chip accommodation kit shown in FIG. 9;
FIG. 12 is a sectional view taken along the line I-I of FIG. 10.
FIG. 13 is a top view of a chip accommodation tool of the chip accommodation kit shown in FIG. 9;
FIG. 14 is a perspective view of the chip accommodation tool of the chip accommodation kit shown in FIG. 9;
FIG. 15 is a bottom view of the chip accommodation tool of the chip accommodation kit shown in FIG. 9;
FIG. 16 is a sectional view taken along the line of II-II of FIG. 13;
FIGs. 17A to 17D are explanatory plan views of a timer unit of the puncture device shown in FIG. 1;

FIGs. 18A to 18C are explanatory views of a bottom and both sides of a timer unit of the puncture device shown in FIG. 1;

FIG. 19 is a view explaining a state in which the timer unit is mounted on a main body;

FIG. 20 is an explanatory view showing a state before the fine needle chip is mounted on the array chuck;

FIG. 21 is an explanatory view showing a state in which the array chuck mounted with a fine needle chip is moved to an ejectable position;

FIGs. 22A to 22D are explanatory views showing a mechanism of puncturing in a state in which stress is not applied;

FIGS. 23A to 23C are views explaining an idling-run distance according to the present invention;

FIGs. 24A to 24B are views showing a relationship between skin swell and strike position;

FIG. 25 is a view showing a relationship between an idling-run distance and a puncture speed;

FIG. 26 is a view showing a relationship between a puncture speed and glucose permeability or a degree of feeling pain; and

FIG. 27 is a block diagram of the timer unit.

## DETAILED DESCRIPTION OF THE PREFERRED EMBODIMENTS

[Overall configuration of puncture device]

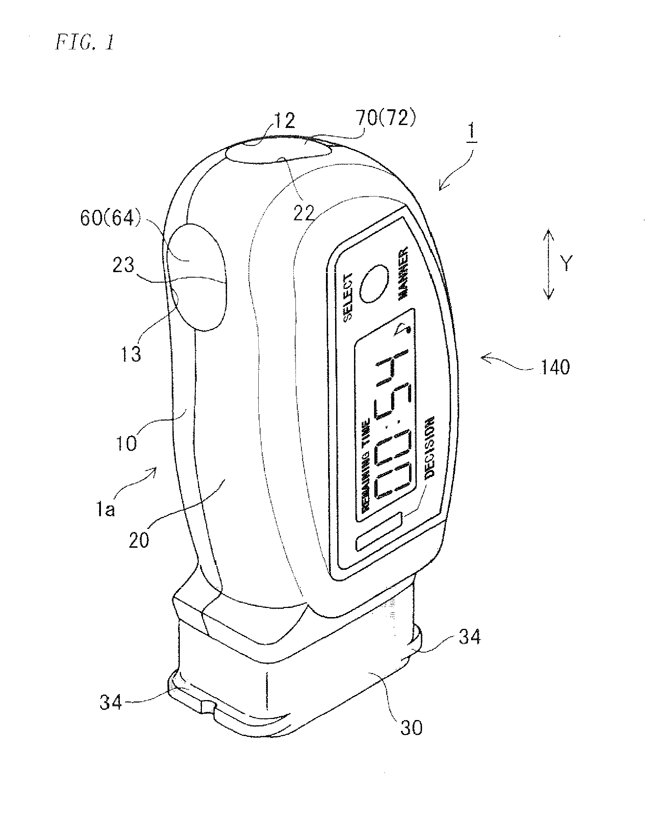

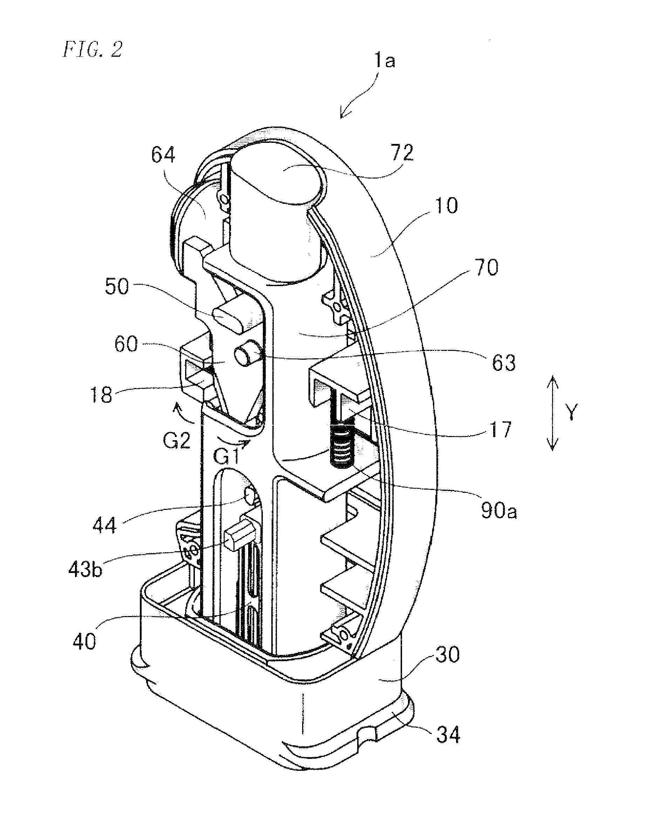

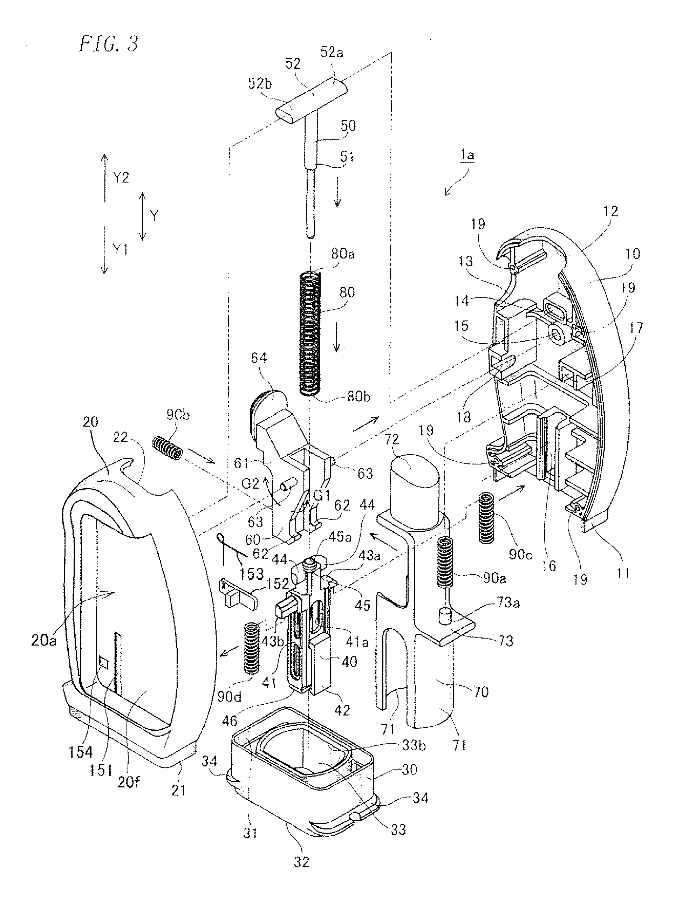

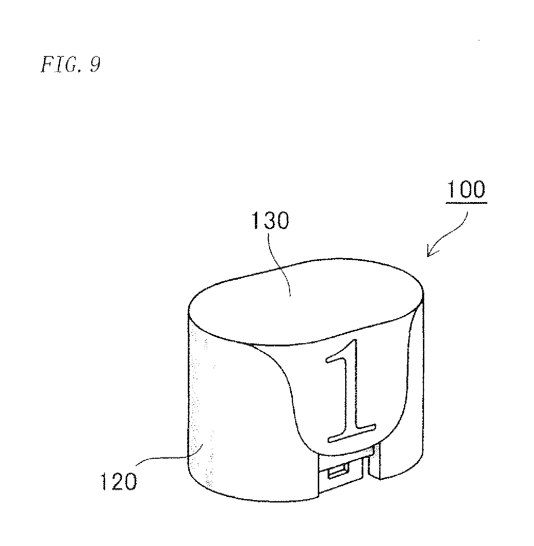

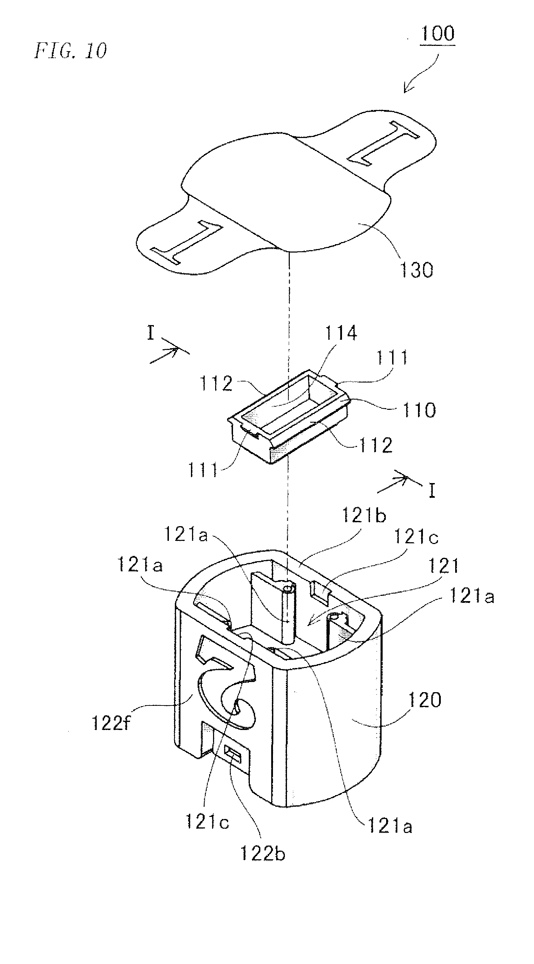

FIG. 1 is a perspective view showing an overall configuration of a puncture device 1 according to an embodiment of the present invention. FIGS. 2 to 8 are views for explaining detailed configuration of respective members of the puncture device 1 shown in FIG. 1. FIG. 9 is a perspective view showing an overall configuration of a chip accommodation kit provided with a fine needle chip to be mounted on the puncture device shown in FIG. 1. FIGs. 10 to 16 are views for showing a detailed configuration of respective members of the chip accommodation kit shown in FIG. 9. Here, although the timer unit is mounted on the main body in FIG. 1, the timer unit is removed from the main body for easy understanding in FIG. 3.

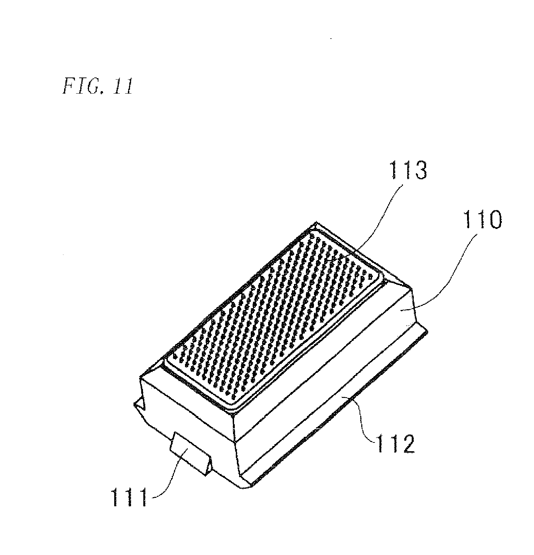

A puncture device 1 (Refer to FIG. 1) according to an embodiment of the present invention is mounted with a fine needle chip 110 (Refer to FIG. 11) which is sterilized and forms extraction pores (fine pores) for extracting body fluid on the subject's skin by contacting a fine needle 113a of the fine needle chip 110 with the subject's skin. Then, the body fluid (tissue fluid) exudated from the extraction pores on the subject's skin which is formed by the puncture device 1 and the fine needle chip 110 is collected in an extraction medium and this extraction medium is measured with a glucose concentration analysis device for calculating the glucose concentration in the tissue fluid. Diabetes patients themselves predict AUC based on the value and monitor and manage a predicted AUC. First, with reference to FIGS. 1 to 12, a configuration of the puncture device 1 according to an embodiment of the present invention is described in detail.

The puncture device 1 forms plural fine extraction pores which penetrate a stratum corneum of epidermis of the skin and do not reach up to vascular plexus in a dermis, and exudates a tissue fluid from the extraction pores. This puncture device 1 comprises a main body 1a having a puncture mechanism for puncturing a subject's skin, and a timer unit 140 having a timer function described below. As shown in FIGs. 1 to 3, the main body 1a of the puncture device 1 comprises a rear cover 10, a front cover 20, a chip accommodation tool insertion member 30, an array chuck 40, a spring stopper 50, a release button 60, an ejector 70, a mainspring 80 (Refer to FIG. 3), and plural springs 90a to 90d (Refer to FIG. 3). Here, seven members (rear cover 10, front cover 20, chip accommodation tool insertion member 30, array chuck 40, spring stopper 50, release button 60, and ejector 70) except for the springs (mainspring 80 and plural springs 90a to 90d) are respectively made of synthetic resin. The puncture mechanism of the main body 1a of the puncture device 1 is principally configured by the array chuck 40, the spring stopper 50, the release button 60, and the mainspring 80. Further, the array chuck 40 configures a piston and the mainspring 80 configures a drive spring. Further, the array chuck 40 has a passage for inserting the spring stopper 50 inside the array chuck 40. The springs 90c and 90d configure a repulsion spring described below.

[Configuration of respective elements of main body]

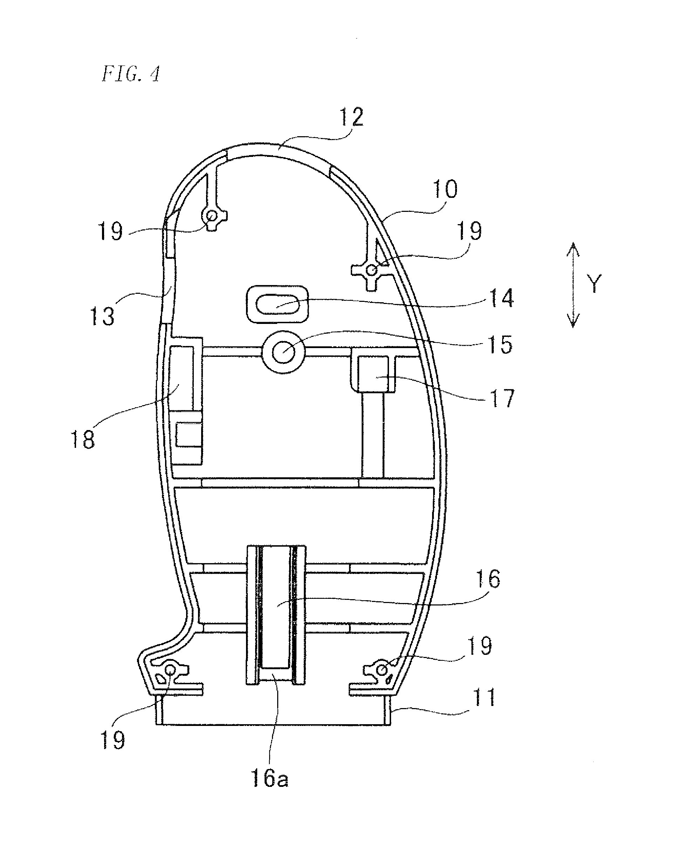

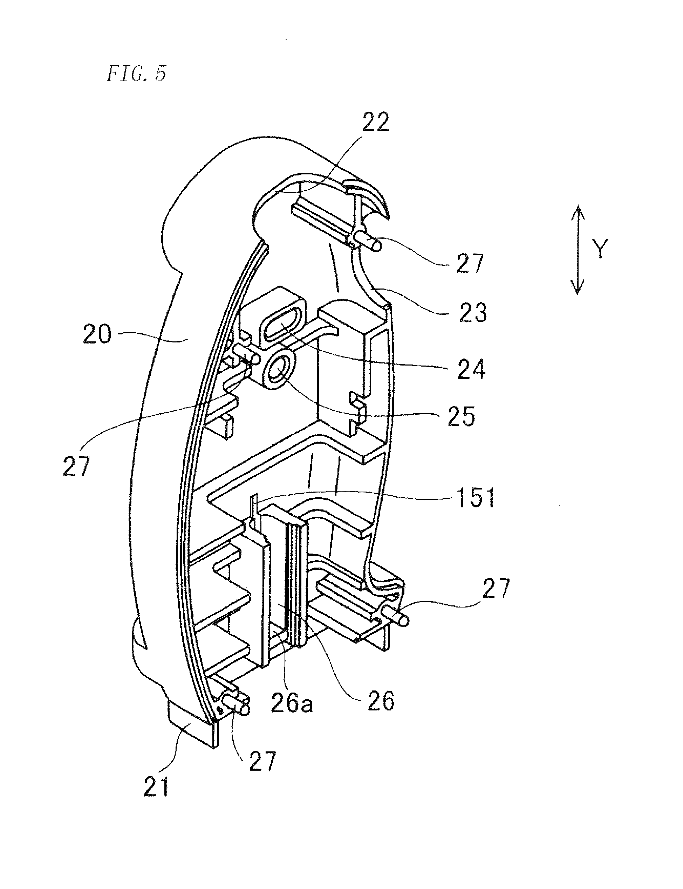

As shown in FIGs. 2 and 3, a housing consisting of the rear cover 10 and the front cover 20 is capable of accommodating therein the array chuck 40, the spring stopper 50, the release button 60, and the ejector 70, the mainspring 80, and the plural springs 90a to 90d. A fitting section 11 is formed on a lower part of the rear cover 10 for fitting the chip accommodation tool insertion member 30, as shown in FIGs. 3 and 4. Further, on an upper part of the rear cover 10, an opening 12 is formed for exposing a button section 72 of the ejector 70 so that the user can press. Further, on a side of the rear cover 10, an opening 13 is formed for exposing a button section 64 of the release button 60. Further, inside the rear cover 10, a concave 14 in which one end 52a of a spring contact section 52 of the spring stopper 50 is fit, a concave 15 in which a support shaft 63 of the release button 60 is engaged, a guide groove 16 for guiding a guide section 43 of the array chuck 40 which moves inside the housing in Y direction (vertical direction in FIGs. 1 to 5), spring installation sections 17 and 18 for installing the springs 90a and 90b respectively, and four pieces of boss insertion pores 19 in which four pieces of boss sections 27 of the front cover 20 are inserted (Refer to FIG. 5) are provided. Further, the spring 90c is installed in the guide groove 16. Here, according to the present embodiment, one end of the mainspring 80 is arranged and retained in the housing by the spring contact section 52 of the spring stopper 50. Therefore, a housing-side contact section is equal to the spring contact section 52.

As shown in FIGs. 3 and 5, and similarly to the rear cover 10, the front cover 20 comprise a fitting section 21 for fitting the chip accommodation tool insertion member 30, an opening 22 for exposing the button section 72 of the ejector 70 so that the user can press, an opening 23 for exposing the button section 64 of the release button 60, a concave 24 in which other end 52b of the spring contact section 52 of the spring stopper 50 is fitted, a concave 25 in which the support shaft 63 of the release button 60 is engaged, and a guide groove 26 for guiding the guide section 43 of the array chuck 40 moving inside the housing in Y direction. Further, the guide groove 26 (Refer to FIG. 5) is provided with the spring 90d (Refer to FIG. 3). Further, in the front cover 20, four pieces of the boss sections 27 are formed in a position opposite to four pieces of the boss insertion pores 19 of the rear cover 10 (Refer to FIG. 3). Therefore, four pieces of the boss sections 27 of the front cover 20 are inserted in four pieces of the boss insertion pores 19 of the rear cover 10, so that the front cover 20 is fit to the rear cover 10 in a positioning state.

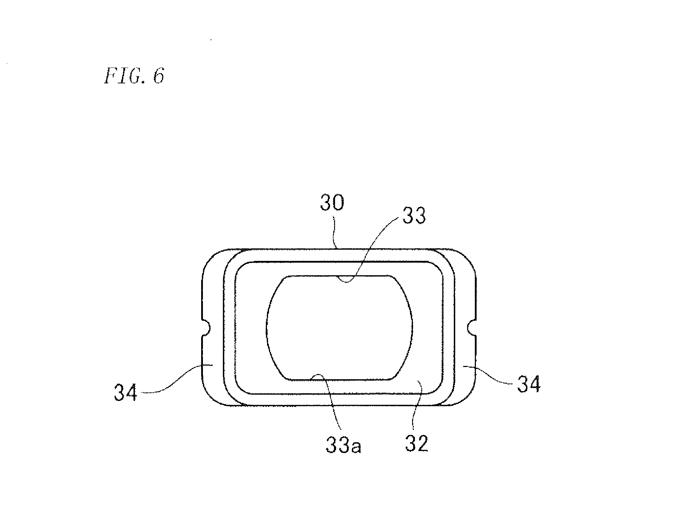

The chip accommodation tool insertion member 30 is provided for inserting a chip accommodation tool 120 accommodating the fine needle chip 110 (Refer to FIG. 11) when the fine needle chip 110 is mounted, and for inserting an empty chip accommodation tool 120 when the fine needle chip 110 which has been already used is disposed of. As shown in FIGs. 3 and 6, this chip accommodation tool insertion member 30 includes a fitting section 31 which is fitted on the fitting section 11 of the rear cover 10 and the fitting section 21 of the front cover 20 (Refer to FIG. 3), a contact surface 32 to be in contact with the skin of subject's arm, a through-hole 33 which has an opening 33a (Refer to FIG. 6) formed on the contact surface 32 and an opening 33b (Refer to FIG. 3) formed on the other side of the opening 33a, and two pieces of flange portions 34 formed so that they project outward from lateral outside surfaces.

Further, according to the present embodiment, the opening 33a formed on the contact surface 32 is configured so that the chip accommodation tool 120 for removably accommodating the fine needle chip 110 (Refer to FIG. 10) is insertable. Therefore, the chip accommodation tool 120 passing through the opening 33a can move through the through-hole 33 in Y direction.

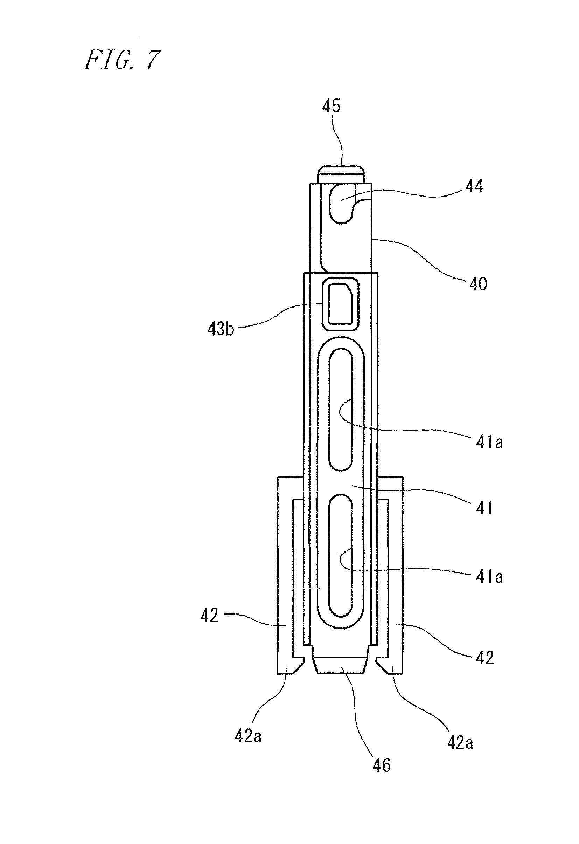

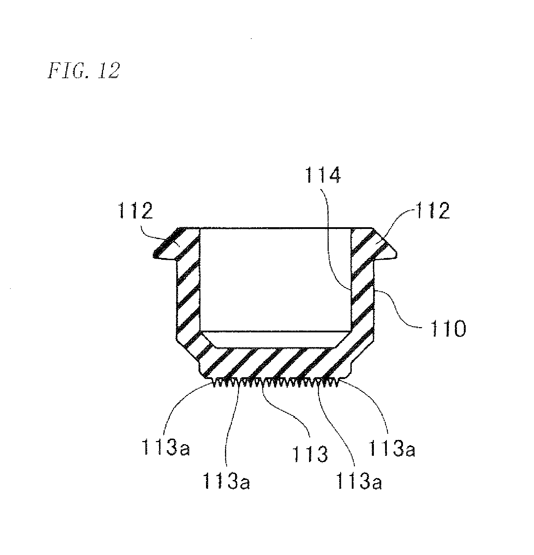

The array chuck 40 which functions as a piston for striking or contacting the fine needle chip 110 to or with the subject's skin is configured so that the array chuck 40 is capable of moving in Y direction along the guide groove 16 of the rear cover 10 and the guide groove 26 of the front cover 20. The fine needle chip 110 (Refer to FIG. 11) retained by the array chuck 40 is capable of moving in Y direction through the through-hole 33 of the chip accommodation tool insertion member 30. As shown in FIGs. 3 and 7, this array chuck 40 includes a body 41 which is provided with plural pores 41a for a purpose of lightweight, a pair of chuck sections 42 which are elastic and deformable and retain the fine needle chip 110 in engagement with a flange portion 112 (Refer to FIG. 12) of the fine needle chip 110, a guide section 43a inserted in the guide groove 16 of the rear cover 10 and a guide section 43b which is inserted in the guide groove 26 of the front cover 20 and has a tip end 43d projecting from a slit 151 described below, two pieces of engagement sections 44 in engagement with two pieces of lock sections 62 of the release button 60 described below, a convex 45 which has an insertion hole 45a (Refer to FIG. 3) capable of inserting a shaft section 51 of the spring stopper 50 described below, and a bush section 46 which is formed in the lower part of the body 41 (in a side of arrow mark Y1). Further, the tip end 42a in contact with the flange portion 112 of the fine needle chip 110 of the chuck section 42 is formed in a taper shape and formed in a hook shape so that the tip end 42a is capable of engaging with the flange portion 112. Further, the guide section 43a is formed so that the guide section 43a contacts with one end of the spring 90c arranged in the guide groove 16 of the rear cover 10, and the guide section 43b is formed so that the guide section 43b contacts with one end of the spring 90d arranged in the guide groove 26 of the front cover 20. Here, the other ends of the springs 90c and 90d are arranged in the guide groove 16 or the guide grove 26 so that they contact inner surfaces of the walls 26a and 26a (Refer to FIG. 4 or 5) which define respective lower ends of the guide groove 16 or the guide groove 26. In other words, in the springs 90c and 90d which configure repulsion springs in the present embodiment, any ends are not locked by any other members.

Here, according to the present embodiment, in a case where two pieces of engagement sections 44 are not engaged with two pieces of lock sections 62 of a release button 60 described below, the array chuck 40 is so configured that the fine needle chip 110 accommodated in the chip accommodation tool 120 is automatically retained by inserting the chip accommodation tool 120 (Refer to FIG. 10) in the opening 33a of the chip accommodation tool insertion member 30. Further, after retaining the fine needle chip 110, the array chuck 40 movable in Y direction is moved in a direction of arrow mark Y2 until the engagement section 44 is locked to the lock section 62.

Further, according to the present embodiment, in a case where two pieces of the engagement sections 44 are not engaged with two pieces of the lock sections 62 of the release button 60 described below, the fine needle chip 110 retained by the array chuck 40 is so configured that the fine needle chip 110 is automatically removed from the chuck section 42 of the array chuck 40 by inserting the chip accommodation tool 120 in the opening 33a of the chip accommodation tool insertion member 30.

Further, according to the present embodiment, the chuck section 42 is integrally formed with other sections (body 41, guide section 43a, 43b, engagement section 44, convex 45, and bush section 46) and all are made of synthetic resin.

The spring stopper 50 is provided for supporting the mainspring 80 which biases the array chuck 40 in a direction of arrow mark Y1. This spring stopper 50, as shown in FIG. 3, includes a shaft section 51 to be inserted in the mainspring 80 and a spring contact section 52 for preventing the mainspring 80 to be inserted in the shaft section 51 from escaping upward (in a direction of arrow mark Y2). Then, an end 52a on one side of the spring contact section 52 and an end 52b on other side thereof are formed so that they are respectively fitted in the concave 14 of the rear cover 10 and the concave 24 of the front cover 20 (Refer to FIG. 5).

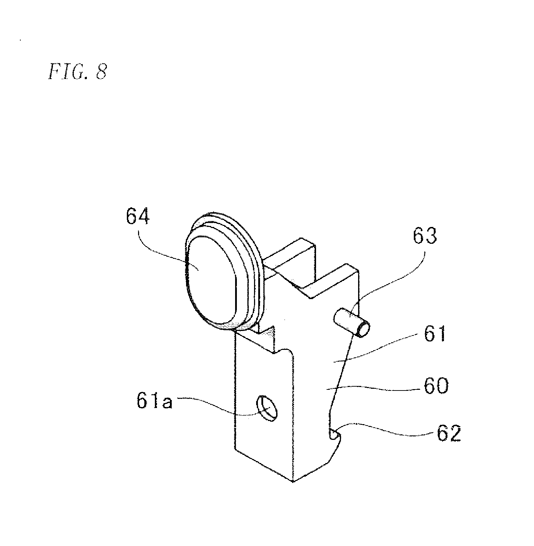

The release button 60, as shown in FIGs. 3 and 8, comprises a body 61, two pieces of the lock sections 62 which engage with two pieces of the engagement sections 44 of the array chuck 40, two pieces of the support shafts 63 which engage with the concave 15 of the rear cover 10 and the concave 25 of the front cover 20 (Refer to FIG. 5), and the button section 64 which is exposed from the opening 13 arranged in a side surface of the rear cover 10 and the opening 23 arranged in a side surface of the front cover 20 (Refer to FIG. 5). Further, a concave 61a to be in contact with one end of the spring 90b (Refer to FIG. 3) which is installed in the spring installation section 18 (Refer to FIGs. 3 and 4) of the rear cover 10 is formed in a side surface having the button section 64 of the body 61 thereon as shown in FIG. 8. Further, according to the present embodiment, two pieces of lock sections 62 have a function of locking the array chuck 40 which is moved in a direction of arrow mark Y2 against a bias force in a direction of arrow mark Y1 of a mainspring 80 described below. In other words, two pieces of lock sections 62 have a function as a stopper for maintaining the array chuck 40 in an ejection standby position.

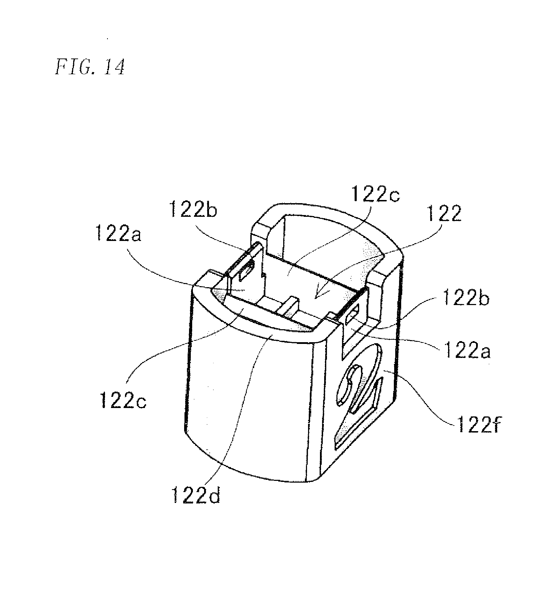

Further, according to the present embodiment, the ejector 70 has a function of discharging the chip accommodation tool 120 accommodating the fine needle chip 110 through the through-hole 33 (Refer to FIG. 3) of the chip accommodation tool insertion member 30. This ejector 70, as shown in FIG. 3, comprises a press section 71 which presses an edge 121b (Refer to FIG. 10) and an edge 122d (Refer to FIG. 14) of the chip accommodation tool 120 which are described below, a button section 72 which is exposed from the opening 12 of the rear cover 10 and the opening 22 of the front cover 20 and can be pressed by the subject, and a contact section 73 in contact with one edge of the spring 90a which is installed in the spring installation section 17 of the rear cover 10. A boss section 73a which is inserted inside the spring 90a is formed in the contact section 73 so that it is possible to prevent release of the spring 90a from the spring installation section 17 of the rear cover 10.

The mainspring 80 is provided for biasing the array chuck 40 in a direction of arrow mark Y1. The shaft section 51 of the spring stopper 50 is inserted inside the mainspring 80 as shown in FIG. 3. Here, the one end 80a of the mainspring 80 is in contact with the spring contact section 52 of the spring stopper 50 and the other end 80b is in contact with upper surface of the engagement section 44 of the array chuck 40. In other words, the mainspring 80 being the drive spring in the present embodiment is in a free state in which the any ends are not locked by any other members.

The spring 90a which is installed in the spring installation section 17 of the rear cover 10 and inserted in the boss section 73a of the contact section 73 of the ejector 70 has a function of biasing the ejector 70, which is pressed up in a direction of arrow mark Y2, in a direction of arrow mark Y1 as shown in FIG. 3. Further, the spring 90b which is arranged in the spring installation section 18 of the rear cover 10 and in the concave 61a (Refer to FIG. 8) of the release button 60 is provided for turning in a direction of arrow mark G1 the release button 60 which is turned around the support shaft 63 as a support point in a direction of arrow mark G2. Further, the springs 90c and 90d which are installed in the guide groove 16 of the rear cover 10 and the guide groove 26 (Refer to FIG. 5) of the front cover 20 have a function of pressing back in a direction of arrow mark Y2 the array chuck 40 which is moved in a direction of arrow mark Y1 due to a bias force of the mainspring 80. Thus, it is possible to prevent the array chuck 40 which moves in a direction of arrow mark Y1 from moving lower than the specific position (in a direction of arrow mark Y1) and it is possible to absorb impact applied to the front cover 10 and the rear cover 20 when the array chuck 40 is ejected.

[Timer unit]

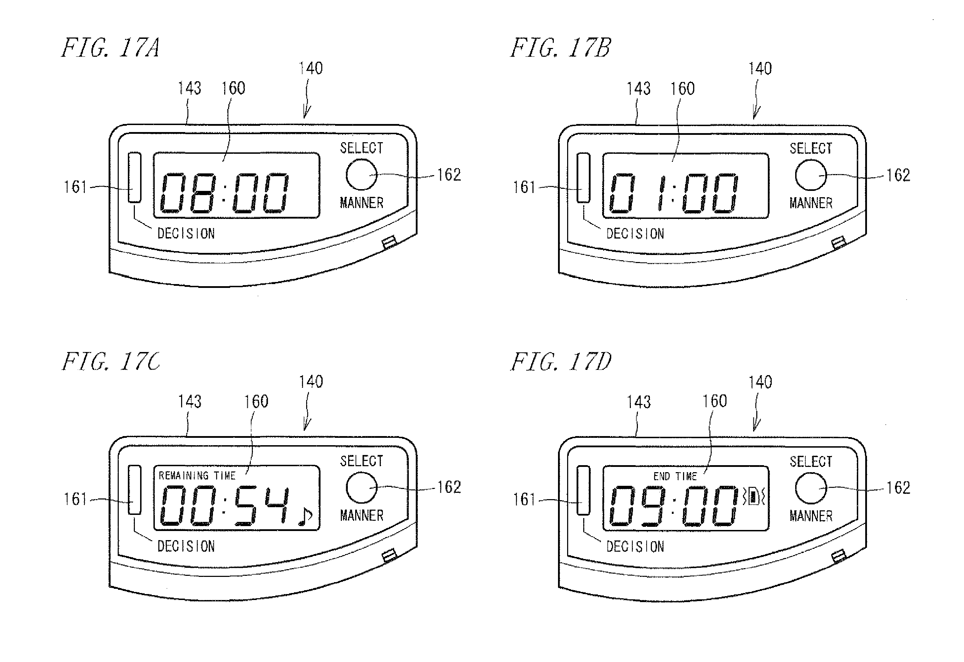

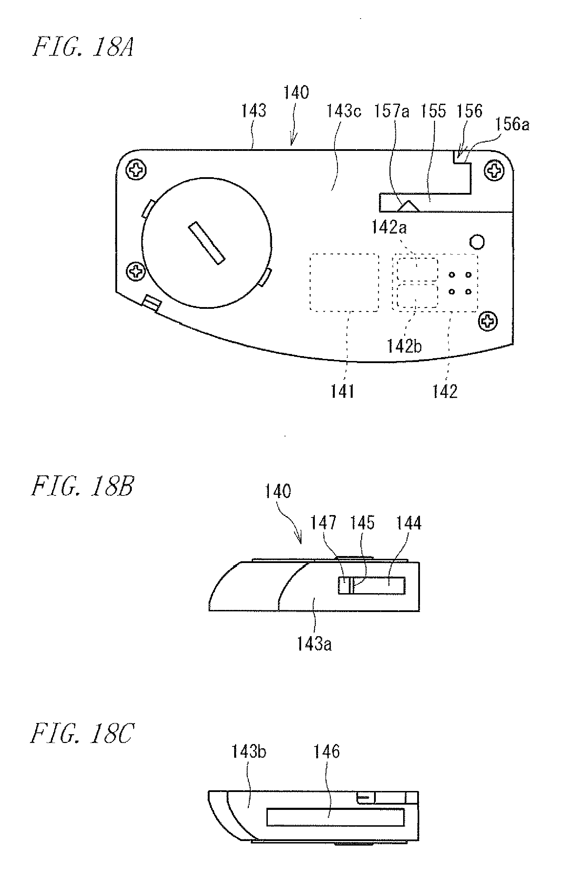

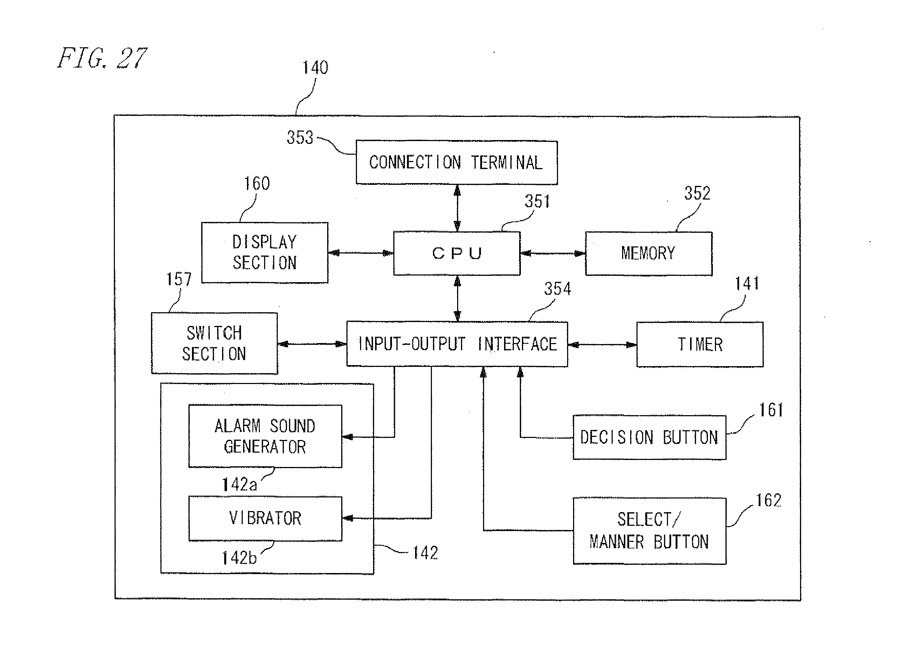

FIGs. 17A to 17D are explanatory plan views of a timer unit 140 in the puncture device 1 according to the present embodiment. FIG. 18A is an explanatory bottom view of the same, FIG. 18B is an explanatory upper view of the same, and FIG. 18C is an explanatory lower view of the same. Further, FIG. 27 is a block diagram of the timer unit 140. As shown in FIG. 27, the timer unit 140 comprises a timer 141, an alarm 142, a switch section 157, a display section 160, a decision button 161, a select/manner button 162, a CPU 351, a memory 352, a connection terminal 353, an input-output interface 354, and the like. The timer unit 140 is covered with a casing 143 made of synthetic resin. The timer 141 has a function of starting measurement of a specific time by a puncture action as described below. The alarm 142 has a function of notifying the subject that the specific time has passed. The CPU 351 is caused to control actions of respective types of components of the timer unit 140. According to the present embodiment, an alarm sound generator 142a emitting sound and a vibrator 142b emitting vibration are provided as the alarm 142, and at least one of them is caused to function by operation of the decision button 161 and the select/manner button 162.

Further, the user operates the decision button 161 and the select/manner button 162 for causing the CPU 351 to adjust time of the timer 141, set extraction time, and select a notifying method through the input-output interface 354. For example, according to the present embodiment, when the decision button 161 is pressed down in a state in which the timer unit 140 is not mounted on the main body 1a, a screen displays time as shown in FIG. 17A and a portion displaying "hour" blinks. When the select/manner button 162 is pressed down in this state, it is possible to change hour display. Further, when the decision button 161 is pressed down in a state that the portion of "hour" blinks, a portion displaying "minute" blinks. When the select/manner button 162 is pressed down in this state, it is possible to change minute display.

When the decision button 161 is pressed down in a state in which the portion displaying "minute" blinks, the screen displays extraction time as shown in FIG. 17B and the extraction time blinks. When the select/manner button 162 is pressed down in this state, it is possible to change the extraction time every 10 minutes. Further, when the decision button 161 is pressed down during blinking of the extraction time, it is possible to lock the extraction time and turn off a power supply of the display section 160. Thus, preparation for activating the timer is completed.

Next, when the array chuck 40 is ejectably loaded as described below, the power supply of the display section 160 is turned on, and the extraction time set up by the user is displayed on the display section 160. Subsequently, when the array chuck 40 is ejected, "remaining time" is displayed on the display section 160 as shown in FIG. 17C. Further, when the select/manner button 162 is pressed down for short time in this state, it is possible to shift the display section 160 to a mode displaying "end time" as shown in FIG. 17D. Further, when the select/manner button 162 is pressed down for long time in a state in which "remaining time" or "end time" is displayed, it is possible to select a method of notifying the subject of end of the extraction time, by sound or vibration, or both of them. Then, which notifying method is selected is displayed by a symbol mark on the display section 160. FIG. 17C shows that sound is selected as a notifying method and FIG. 17D shows that vibration is selected as a notifying method. Further, when the decision button 161 or the select/manner button 162 is pressed down in a state in which extraction time ends and the alarm 142 is activated it is possible to stop the sound or the vibration which is emitted, or both of them.

The timer unit 140 is provided with the memory 352 which memorizes variety of information related to the subject and measurement. The memory 352 is composed of ROM and RAM. As the variety of information memorized by the memory 352, examples are a name of the subject (patient) and a lot and a type of gel being an extraction medium. The timer unit 140 is provided with the connection terminal 353 for transferring these types of information from the timer unit 140 to the measurement device or PC (personal computer) . In a case where the puncture device of the present invention is utilized in a medical institution, the subject carries around the timer unit 140 with a gel reservoir member (collection member) for extraction being applied to the puncture site of the subject. A component subject to be measured in the extracted tissue fluid is measured, after the timer unit 140 and the gel reservoir member in which the tissue fluid is extracted and retained are collected after the specific extraction time has passed. Here, it is possible for a measurer to obtain information about the patient being the subject only by receiving the timer unit 140 and the gel reservoir member for extraction, so that work such as recording the variety of information by the measurer is not required.

Further, when extraction time and measurement date and time are memorized by the memory 352 of the timer unit 140, an individual is not required to record separately, and therefore convenience improves. Further, it is also possible to cause time when the subject has a meal to be memorized. It is possible to review it when the obtained data are analyzed by recording meal time when puncture is performed and measurement starts after the meal or history of meal time.

Further, it is possible to cause the timer unit 140 to record a past blood glucose level of the subject which is obtained by the seif-monitoring of blood glucose (SMBG) and it is possible to consider it together with a result of AUC measurement which is currently obtained. Especially, in the case of the SMBG result which is measured in combination with the AUC measurement, the result is possible to be applied to AUC wave analysis.

It is also possible to display these outputs from the timer unit 140 on the display section 160 together with measurement time and it is possible to output the outputs to PC for data analysis from the connection terminal 353 for PC provided in the timer unit 140.

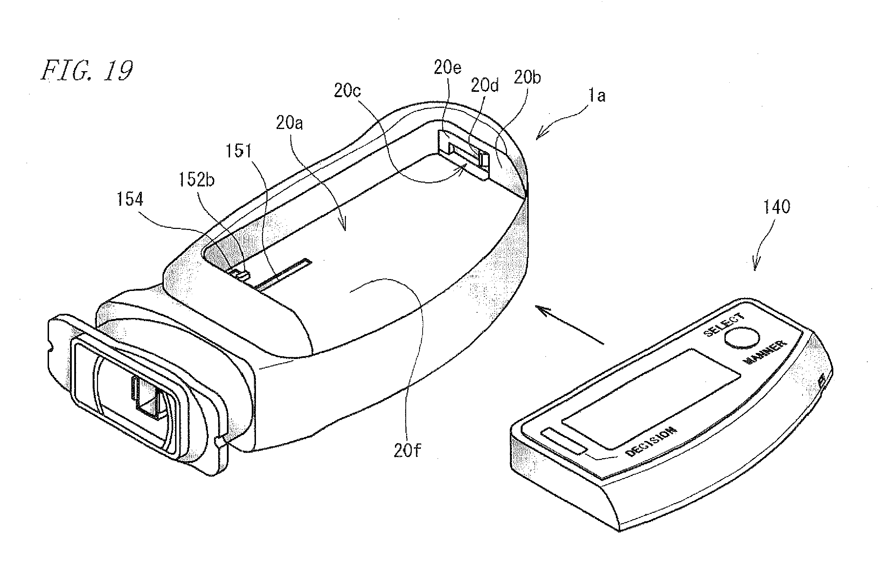

The timer unit 140 is removably mounted on the main body 1a as shown in FIG. 19. More particularly, a concave 20a having a shape and a size corresponding to outline of the timer unit 140 is formed in the front cover 20. When the timer unit 140 is mounted so that it fits inside the concave 20a of the front cover 20, obtained outline of the puncture device 1 is substantially continuous and even as shown in FIG. 1. As shown in FIG. 19, an opening 20c is formed in an upper side wall 20b which defines the concave 20a of the front cover 20. An engagement piece 20e is arranged inside the opening 20c, and an engagement nail 20d projecting outward from the opening 20c is arranged on the tip end of the engagement piece 20e. This engagement piece 20e is a cantilever beam with an end on the side of the engagement nail 20d being a free end and the engagement piece 20e is swingable within a specific range with a root part thereof as a basic point.

When the timer unit 140 is mounted on the main body 1a, as shown in FIG. 18B, a guide groove 144 for guiding the engagement nail 20d of the engagement piece 20e is formed in one side surface 143a (side surface located upper side in use of the puncture device 1 with the timer unit 140 being mounted on the main body 1a) of a casing 143. A convex line 145 perpendicular to the longitudinal direction of the guide groove 144 is formed in depth of the guide groove 144 (left side in FIG. 18B).

Further, a guide groove 146 for guiding a rib which is formed in a lower side wall defining the concave 20a is formed in other side surface 143b which faces the one side surface 143a of the casing 143.

In a case where the timer unit 140 having the above-mentioned configuration is mounted on the main body 1a so that the timer unit 140 is positioned inside the concave 20a of the front cover 20, the engagement nail 20d of the engagement piece 20e moves in the guide groove 144 in the one side surface 143a of the casing 143, and the rib formed in the lower side wall moves in the guide groove 146 in the other side surface 143b of the casing 143. Here, the engagement nail 20d of the engagement piece 20e moves in the guide groove 144 in contact with the bottom surface 144a of the guide groove 144, and passes over the convex line 145, and is engaged with an engagement concave 147. Mount of the timer unit 140 on the main body 1a is completed by engagement between the engagement nail 20d and the engagement concave 147, and it prevents the timer unit 140 from being removed from the main body 1a due to contact and the like.

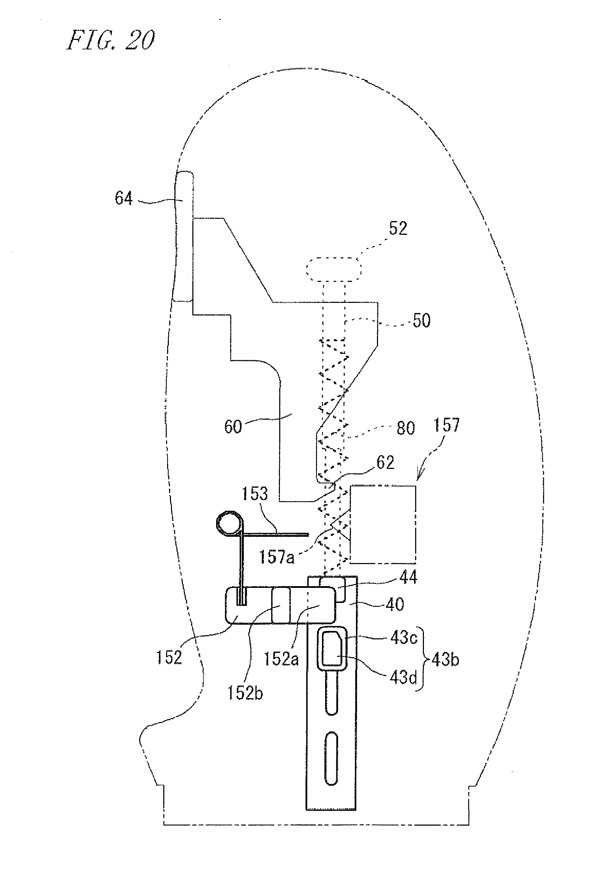

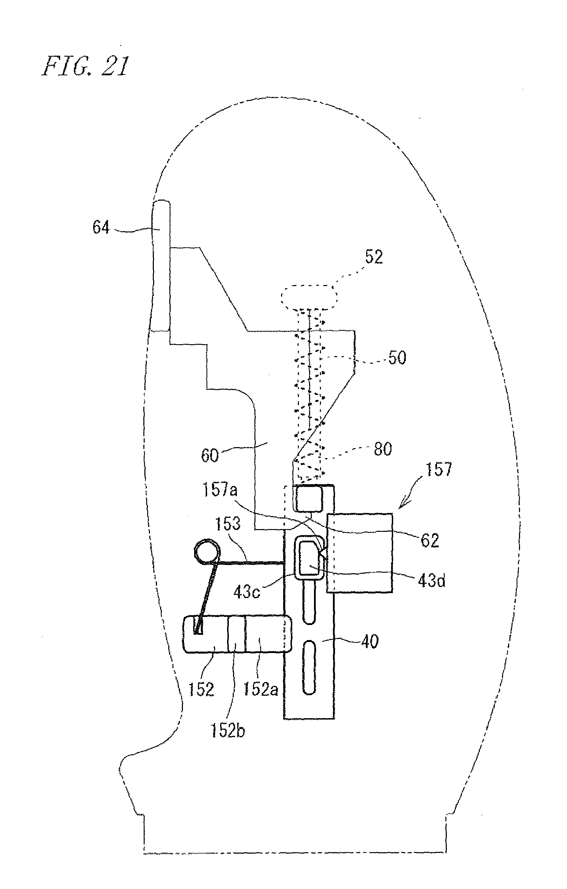

FIG. 20 is an explanatory view showing a state before the fine needle chip is mounted on the array chuck 40. FIG. 21 is an explanatory view showing a state in which the array chuck 40 mounted with the fine needle chip is moved to an ejectable position. FIGs. 20 and 21 show arrangement of the array chuck 40 and the like in the puncture device 1, which is viewed from the side of the timer unit 140, that is, the side of the front cover 20. Here, illustration of the fine needle chip is omitted in FIGs. 20 and 21 for easy understanding. Further, because the timer unit 140 is not mounted on the main body 1a in the state shown in FIG. 20, the switch section 157 in the timer unit 140 described below is drawn by an imaginary line (two dot chain line). Further, the spring stopper 50, the engagement section 44, the release button 60, and the mainspring 80 are drawn by an imaginary line (two dot chain line).

As shown in FIGs. 20 and 21, the guide section 43b is projectively provided in the upper end of one side surface (facing the timer unit 140 mounted on the main body 1a) of the array chuck 40 being a piston section. This guide section 43b comprises a basic section 43c locked to the one side surface and a tip end 43d(first projection) which is integrally formed with the basic section 43c and thinner than the basic section 43c. The tip end 43d of the guide section 43b projects outward from the slit 151 which is formed in a bottom wall 20f defining the concave 20a of the front cover 20 (Refer to FIGs. 3 and 19).

Further, an engagement piece 152 which is movable is arranged in the housing configured by the front cover 20 and the rear cover 10. This engagement piece 152 has a second projection 152a and a third projection 152b which projects in a perpendicular direction to a projection direction of the second projection 152a. The engagement piece 152 is biased by a coil spring 153 being a bias means which is arranged in the housing in such a direction that the engagement piece 152 is engaged with the tip end 43d of the guide section 43b. The third projection 152b of the engagement piece 152 projects outward from a slit 154 (Refer to FIGs. 3 and 19) formed in the bottom wall 20f, similarly to the tip end 43d of the guide section 43b.

In a state shown in FIG. 20, the timer unit 140 is not mounted on the main body 1a. In this state, the engagement piece 152 proceeds due to a bias force of the coil spring 153 in such direction that the engagement piece 152 engages with the tip end 43d of the guide section 43b. Therefore, even though the array chuck 40 mounted with the fine needle chip 110 is forced to push into the device, it is impossible to push into because the tip end 43d of the guide section 43b contacts with the second projection 152a of the engagement piece 152.

On the other hand, when the timer unit 140 is mounted on the main body 1a as described below, it is possible to mount the fine needle chip 110 on the array chuck 40 and push the array chuck 40 into the device since the engagement piece 152 moves in such a direction that the engagement with the tip end 43d of the guide section 43b is released. As shown in FIG. 21, when the array chuck 40 is pushed into the device, the tip end 43d of the guide section 43b presses the tip end 157a of the switch section 157.

A guide groove 155 being a groove is formed in a position which is on a rear surface or the bottom surface 143c (Refer to FIG. 18A) of the casing 143 of the timer unit 140 and which faces the slit 151 when the timer unit 140 is mounted on the main body 1a. The switch section 157 with the tip end 157a projecting into the guide groove 155 is provided inside the casing 143. The tip end 157a of the switch section 157 is configured so that it can recede from the guide groove 155 by pressure.

Further, a notch 156 (Refer to FIG. 18A) is formed in a side surface which is a side surface of the casing 143 and where the casing 143 is mounted on the main body 1a. The notch 156 is formed in such a position that the bottom surface 156a thereof contacts with the third projection 152b of the engagement piece 152 when the timer unit 140 is mounted on the main body 1a.

[Lock mechanism and turn-on mechanism]

Next, a lock mechanism which inhibits a puncture action while the timer unit is not mounted and a turn-on mechanism which turns on a power supply of the display section 160 by loading the fine needle chip 110 on the array chuck 40 are explained.

In a state in which the timer unit 140 is not mounted on the main body 1a as shown in FIGs. 19 and 20, the array chuck 40 is biased in a direction of puncture by the mainspring 80 as shown in FIG. 3, and the tip end (the first projection) 43d of the guide section 43b of the array chuck 40 projects outward from the slit 151. Further, the engagement piece 152 is biased by the coil spring 153 in such direction that the second projection 152a thereof engages with the tip end 43d. The second projection 152a of the engagement piece 152 is located on an upper side of the tip end 43d, that is, in depth side or inner side (in Y2, direction) of the array chuck 40 with the tip end 43d being as a basis. Therefore, in this state, it is impossible to push the array chuck 40 up to a position that the fine needle chip 110 is mounted on the array chuck 40 and the fine needle chip 110 can be ejected.

When the timer unit 140 is mounted on the main body 1a, the bottom surface 156a of the notch 156 which is formed in a side wall of the casing of the timer unit 140 contacts with the third projection 152b of the engagement piece 152, and moves the engagement piece 152 in such a direction that the second projection 152a recedes from the tip end 43d against bias force of the coil spring 153. Therefore, since engagement between the tip end 43d of the array chuck 40 and the second projection 152a of the engagement piece 152 is released (lock released), it is possible that the array chuck 40 moves opposite to a puncture direction.

When the fine needle chip 110 is mounted on the array chuck 40 and the array chuck 40 is pushed into the device opposite to a puncture direction after the timer unit 140 is mounted on the main body 1a, the tip end 43d as a press member moves in the guide groove 155 of the casing 143 of the timer unit 140, and the tip end 43d presses the tip end 157a of the switch section 157, and the tip end 43d retreats the tip end 157a from inside of the guide groove 155. According to the present embodiment, the power supply of the display section 160 is turned on by pressure of the switch section 157 by the tip end 43d as a press member, and the extraction time set up by the user is displayed on the display section 160. More particularly, when the CPU 351 recognizes the pressure of the switch section 157 through the input-output interface 354, the CPU 351 can turn on the power supply of the display section 160. Here, the power supply of the display section 160 is turned off after a given time has passed.

Next, when the array chuck 40 is ejected by pressing the button section 64 of the release button 60, engagement between the tip end 43d of the array chuck 40 and the tip end 157a of the switch section 157 is released, and the tip end 157a of the switch section 157 again projects inside the guide groove 155. According to the present embodiment, the CPU 351 recognizes release of pressure of the tip end 157a or a puncture action through the input-output interface 354 and causes the timer 141 to start time measurement.

[Chip accommodation kit]

Next, with reference to FIGs. 1, 3, 7 and 9 to 16, a chip accommodation kit 100 composed of a fine needle chip 110 which is mounted on the array chuck 40 of the puncture device 1 according to the present embodiment, a chip accommodation tool 120 accommodating the fine needle chip 110, and a sterilization preservation seal 130 are explained in detail.

The fine needle chip 110 is mounted in the array chuck 40 (Refer to FIG. 7) of the above-mentioned puncture device 1 (Refer to FIG. 1) for use and has plural fine needles 113a for forming plural extraction pores to exudate a tissue fluid (body fluid) from the subject's skin. The fine needle chip 110 is formed in a shape of substantial rectangle in a plane view, as shown in FIGs. 10 to 12. The fine needle chip 110 includes a pair of projections 111 which are arranged so as to project outward from lateral outside surfaces, a pair of flange portions 112 which are arranged so as to project outward from longitudinal outside surfaces, a fine needle array section 113 which has 305 pieces of fine needles 113a, and a concave 114 in which the bush section 46 (Refer to FIG. 7) of the array chuck 40 of the puncture device 1 described above is inserted. Further, a pair of projections 111 are formed so that they are engaged by an engagement pore 122b of the chip accommodation tool 120 described later. A pair of flange portions 112 are formed so that they engage the tip end 42a of the chuck section 42 (Refer to FIG. 7) of the array chuck 40. Here, the fine needle chip 110 together with 305 pieces of fine needles 113a are formed of synthetic resin. Now, except for the fine needle chip 110 including the fine needle array section 113 having 305 pieces of the fine needles 113a described above, other fine needle chips such as a fine needle chip including an fine needle array section having 189 pieces of fine needles may be used.

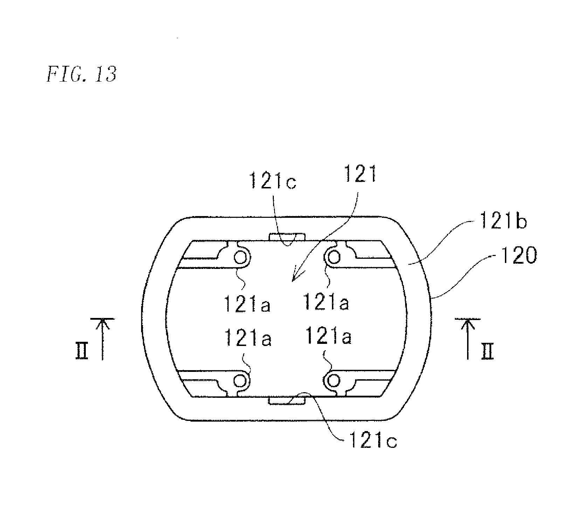

According to the present embodiment, the chip accommodation tool 120 formed of synthetic resin includes an opening 121 for accommodating the fine needle chip 110 (Refer to FIG. 10) before use which is sterilized and an opening 122 for accommodating the fine needle chip 110 after use which is punctured on the subject's skin, as shown in FIGs. 10 and 13 to 16. The opening 121 and the opening 122 are arranged in an opposite side to each other. To the opening 121, the sterilization preservation seal 130 described below is applied for sealing the opening 121 which accommodates the fine needle chip 110 unused. Further, as shown in FIGs. 10 and 13, the opening 121 has four pieces of support sections 121a which support side surfaces of the fine needle chip 110 before use which is sterilized, an edge 121b which contacts with the press section 71 (Refer to FIG. 3) of the ejector 70, and an allowance 121c which is formed so that the projection 111 (Refer to FIGs. 10 and 11) of the fine needle chip 110 retained by the support section 121a does not contact with the edge 121b.

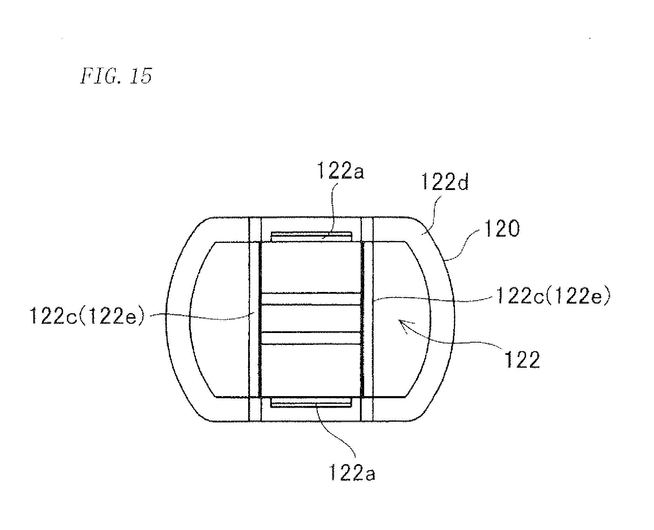

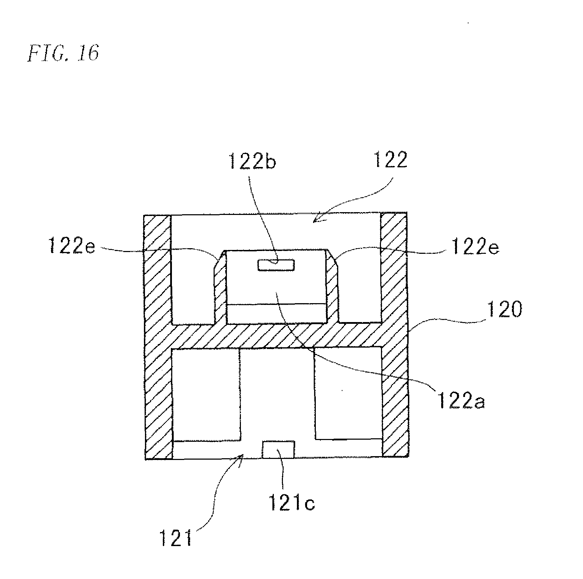

Further, according to the present embodiment, as shown in FIGs. 14 and 15, the opening 122 includes the retention section 122a which has the engagement pore 122b where the projection 111 (Refer to FIGs. 10 and 11) of the fine needle chip 110 which is already used and punctured on the subject's skin is inserted. Further, the opening 122 includes a release piece 122c which releases engagement between the chuck section 42 (Refer to FIG. 7) of the array chuck 40 of the puncture tool 1 and the flange portion 112 of the fine needle chip 110, and the edge 122d which contacts with the press section 71 (Refer to FIG. 3) of the ejector 70. A tip end 122e of the release piece 122c is formed in a taper shape as shown in FIG. 16. Further, a mark "2" is engraved on the side surface 122f of the chip accommodation tool 120 for easy confirmation in a case where the opening 122 is arranged upside.

The sterilization preservation seal 130 is formed of aluminum film and has a function of inhibiting adhesion of viruses, germs, and the like to the fine needle chip 110 which is sterilized by $\gamma$-ray irradiation. The sterilization preservation seal 130 is applied so as to cover the opening 121 which accommodates the fine needle chip 110 before use, as shown in FIGs . 9 and 10. Further, the sterilization preservation seal 130 is applied so as to cover "2" engraved on the side surface 122f of the chip accommodation tool 120 as described above. On a portion applied to the side surface 122f of the chip accommodation tool 120, a mark "1" is engraved for easy confirmation in a case where the opening 121 is arranged upside as shown in FIG. 9.

According to the present embodiment, there is provided the array chuck 40 for retaining the fine needle chip 110 by inserting the chip accommodation tool 120 in the opening 33a of the chip accommodation tool insertion member 30, in a case where engagement between the engagement section 44 of the array chuck 40 and the lock section 62 of the release button 60 is released. Therefore, it is possible that the subject causes the chuck section 42 of the array chuck 40 to retain the flange portion 112 of the fine needle chip 110 only by moving the puncture tool 1 in such way that the chip accommodation tool 120 is inserted in the opening 33a of the chip accommodation tool insertion member 30. Further, the lock section 62 (release button 60) which locks the array chuck 40 by engaging with the engagement section 44 of the array chuck 40 is provided and the array chuck 40 is configured so that it can move in Y direction. Then, it is possible that the fine needle chip 110 is retained in the array chuck 40 and the array chuck 40 is locked by the lock section 62 in a state in which the array chuck 40 is moved in a direction of arrow mark Y2 against a bias force by the mainspring 80. Therefore, the subject can set the puncture device 1 to a lock state in which the array chuck 40 retaining the fine needle chip 110 is biased in a direction toward the subject's skin (direction of arrow mark Y1). Thus, the subject can set to a possible state in which the puncture device 1 can form the fine pores on the subject's skin only by moving the puncture device 1 without requiring troublesome work. Further, by pressing the button section 64 of the release button 60 from this state, engagement between the engagement section 44 of the array chuck 40 and the lock section 62 is released. Then, the fine needle chip 110 can pass through the opening 33a of the chip accommodation tool insertion member 30 and move toward a direction of arrow mark Y1, and the fine pores can be formed at the puncture site of the subject's skin.

Further, according to the present embodiment, when the chip accommodation tool 120 which is empty and does not accommodate the fine needle chip 110 is inserted in the opening 33a of the chip accommodation tool insertion member 30 in a case where the engagement between the engagement section 44 of the array chuck 40 and the lock section 62 is released, it is possible that the subject easily removes the fine needle chip 110 which is already used and retained by the array chuck 40 in a state of engagement release from the lock section 62 only by moving the puncture device 1 so as to insert the chip accommodation tool 120 in the opening 33a of the chip accommodation tool insertion member 30. Therefore, it is possible that the subject safely disposes of the used fine needle chip 110 without touching the used fine needle chip 110.

[Puncture mechanism not subjected to spring stress]

According to the present embodiment, in consideration of presence of variation in swell of the subject's skin, "idling-run interval" of a specific length is set up so as not to change puncture speed depending on strike positions with the skin. This "idling-run interval" is an interval where the array chuck 40 moves without receiving bias force and repulsive force from any one of the springs 90c and 90d and the mainspring 80. It may be considered that the array chuck 40 in this interval moves at a substantially nearly constant speed. Therefore, even though there occurs variation in the skin swell, it is possible to uniform the strike speed of the fine needle chip 110 to the skin, by setting up a length of the interval so that the fine needle chip 110 strikes against the subject's skin in this interval. Therefore, it is possible to prevent occurrence of variation in degree of the fine pore formation.

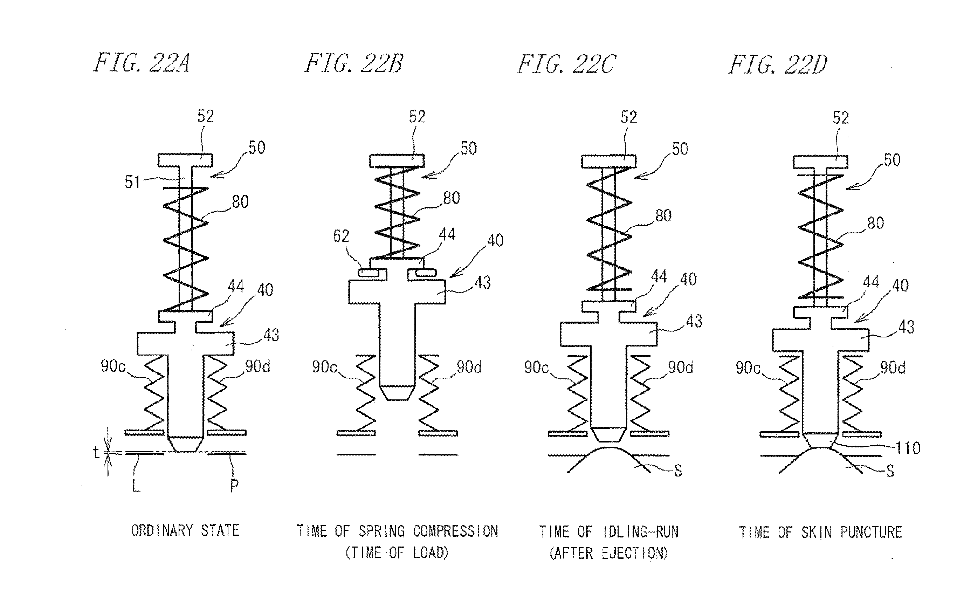

Next, principle of "puncture not subjected to spring stress" is explained with reference to FIGs. 22A to 22D. In FIGs. 22A to 22D, respective elements such as the mainspring 80 are modeled for easy understanding. Further, in FIGs. 22A to 22D, L shows a lowest surface of the puncture device 1. Specifically, L shows a contact surface 32 (Refer to FIG. 6) of the chip accommodation tool insertion member 30 which contacts the skin. P shows "punctured skin surface (supposed strike position)" described below.

FIG. 22A shows a state after the array chuck 40 is ejected, more particularly, a state in which the puncture device 1 separates from the subject's skin after ejection. In this state, the mainspring (drive spring) 80 moves downward by its own weight and a lower end thereof contacts the upper surface of the engagement section 44 of the array chuck 40. Here, the mainspring 80 is inserted through the shaft section 51 of the spring stopper 50. And the mainspring 80 is in a free state in which both ends thereof are not locked by any other members. Further, there is a clearance between the upper end of the mainspring 80 and the lower surface of the spring contact section 52 of the spring stopper 50. The lower surface of the guide section 43 of the array chuck 40 contacts the upper surface of the springs (repulsion springs) 90c and 90d being in a free state in which both ends thereof are not locked by any other members.

FIG. 22B shows a state in which the array chuck 40 mounted with the fine needle chip 110 is pushed inside the device against a bias force of the mainspring 80 and the array chuck 40 is ejectable. In this state, the lock section 62 of the release button 60 engages with the engagement section 44 of the array chuck 40. And thus movement of the array chuck 40 in a puncture direction is inhibited (Refer to FIG. 21).

FIG. 22C shows a state in which the array chuck 40 which is released from engagement with the lock section 62 of the release button 60 by pressure of the button section 64 of the release button 60 is driven in a puncture direction by a bias force of the mainspring 80. More particularly, it shows a state in which the mainspring 80 in a compressed state extends up to its natural length, and subsequently, the array chuck 40 accelerated by the mainspring 80 separates from the mainspring 80, and the guide section 43 of the array chuck 40 does not contact the springs 90c and 90d. In this state, since the array chuck 40 dose not contact any springs and thus does not receive a bias force or a repulsive force of the spring, it may be considered that the array chuck 40 moves at a substantially constant speed. An interval of this movement is an interval when the array chuck 40 moves from a position where engagement between the array chuck 40 and the mainspring 80 is released to a position where the guide section 43 of the array chuck 40 contacts the springs 90c and 90d.

FIG. 22D shows a state in which the fine needle chip 110 mounted on the tip end of the array chuck 40 strikes against the subject's skin S. This strike is carried out in the interval of the movement described above. In this state, the upper end of the mainspring 80 separates from the spring contact section 52, while the lower end of the mainspring 80 separates from the engagement section 44 of the array chuck 40. Therefore, the mainspring 80 does not provide an extension force to the array chuck 40. Further, the array chuck does not receive a repulsive force from the springs 90c and 90d since the guide section 43 of the array chuck 40 does not contact the upper end of the springs 90c and 90d. Here, when the guide section 43 contacts the upper end of the springs 90c and 90d, the springs 90c and 90d start compression.

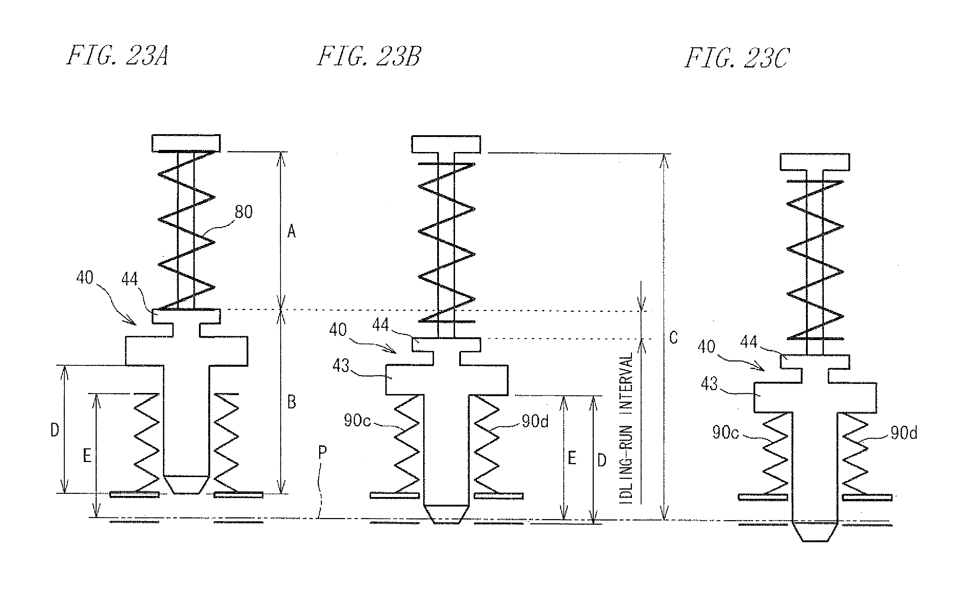

FIGs. 23A to 23C are views explaining "idling-run interval" as described above. FIG. 23A shows a state in which the mainspring 80 contacts the upper surface of the engagement section 44 of the array chuck 40 for causing a drive force to the array chuck 40, and the mainspring 80 extends eventually to the natural length A. After this state of natural length A, engagement between the array chuck 40 and the mainspring 80 is released, and the array chuck 40 separates from the mainspring 80, because a movement speed of the array chuck 40 is higher than an extension speed of the mainspring 80. Here, the extension speed is a speed at which the spring slightly extends based on the natural length A when the spring in a compression state is released.

After the mainspring 80 becomes the natural length A, the array chuck 40 runs (moves) without receiving a force from any one of the springs 80, 90c, and 90d, until the fine needle chip 110 strikes the subject's skin or the lower surface of the guide section 43 of the array chuck 40 contacts the springs 90c and 90d as shown in FIG. 23B. An interval of this running is referred to as "idling-run interval". The array chuck 40 moves substantially without acceleration or deceleration, since the array chuck 40 does not receive a stress from the spring in the "idling-run interval". Here, in a case where, for example, the puncture device 1 is ejected in an idling state without contacting the subject's skin, the array chuck 40 proceeds further the state shown in FIG. 23B to a state in which the springs 90c and 90d are compressed as shown in FIG. 23C. In this case, since the springs 90c and 90d are provided as described above, it is also possible that the array chuck 40 absorbs impact applied to the front cover 10 and the rear cover 20.

In a case of the puncture device not provided with the repulsion spring (case where springs 90c and 90d are omitted in the puncture device in FIGs. 23A to 23C), it is possible to secure the above-mentioned "idling-run interval" by making a length between the tip end of the fine needle chip 110 and a position where the mainspring 80 contacts the spring contact section 52 shorter than a length between the punctured skin surface and the spring contact section 52 when the puncture device is put on the skin, in an extension direction of the mainspring (drive spring). Here, A represents a natural length of the mainspring 80 (drive spring) (Refer to FIG. 23A). B represents a length between a tip end of the fine needle of the fine needle chip 110 and a contact portion of the array chuck 40 which contacts a front-end-side end of the mainspring 80. C represents a length between a back-end-side end of the mainspring 80 in a compressed state and a punctured skin surface P (supposed strike position) which the fine needle chip 110 is supposed to strike against. Then the "idling-run interval" is secured with C > A + B. The larger difference between a length C and a length (A + B) becomes, the longer "idling-run interval" is secured.

Further, in a case of the puncture device provided with the repulsion spring as in the present embodiment, it is possible to secure the "idling-run interval" by making a length between the tip end of the puncture needle and a contact portion with the springs 90c and 90d at the guide section (repulsion spring reception section) 43 receiving the springs 90c and 90d (repulsion spring) longer than a length between the punctured skin surface and a start position where the springs 90c and 90d start compression by the guide section 43, by drive due to extension of the mainspring 80. Here, A represents a natural length of the mainspring (drive spring) 80. B represents a length between a tip end of the fine needle of the fine needle chip 110 and a contact portion of the array chuck 40 which contacts a front-end-side end of the mainspring 80. C represents a length between a back-end-side end of the mainspring 80 in a compressed state and a punctured skin surface P (supposed strike position) which the fine needle chip 110 is supposed to strike against. D represents a length between a front-end-side side surface of the guide section (repulsion spring reception section) 43 receiving the springs 9Dc and 90d (repulsion spring) and a tip end of the fine needle of the fine needle chip 110. E represents a length between a front-end-side side surface of the guide section 43 of the array chuck 40 when the springs 90c and 9Dd start compression and the punctured skin surface P (supposed strike position) described above. Then the "idling-run interval" is secured with C > A + B and D > E. The larger difference between a length C and a length (A + B) becomes, and the larger difference between a length D and a length E becomes, the longer "idling-run interval" is secured.

Here, in the present specification, "punctured skin surface P" is a strike surface between the fine needle chip 110 and the subject' skin, which is supposed in a design of the device, and is a supposed strike position. A distance t between the punctured skin surface P and the contact surface 32 of the puncture device 1 may be set up at 0.2 to 0.8 mm, preferably, approximately 0.5 mm, for example (Refer to FIG. 22A). Because it is not considered that a portion to be punctured is concave when the puncture device 1 is pushed against the subject' s skin, the array chuck 40 is enabled to strike the subject's skin within the idling-run interval where the array chuck 40 is not accelerated and decelerated by supposing such strike surface and setting up length of the above-described A to E based on the supposed surface.

A puncture speed of the fine needle chip 110 on the punctured skin surface P is preferably 4 to 8 m/s, more preferably approximately 6 m/s.

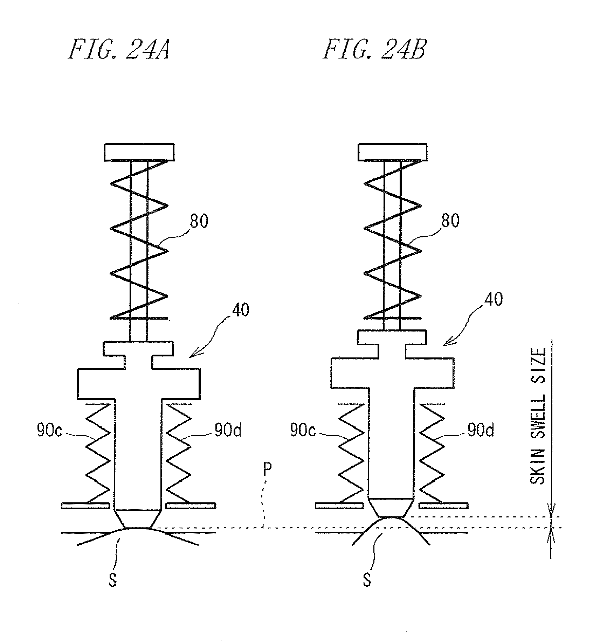

FIGs. 24A to 24B are views showing a relationship between a skin swell and a puncture site. In a case shown in FIG. 24A, swell of the skin S is small and the puncture site is at the supposed punctured skin surface P or slightly above it. In this case, the array chuck 40 separates from the mainspring 80 and in a state immediately before contact with the springs 90c and 90d. In other words, a puncture action is carried out in the "idling-run interval" described above. Meanwhile, in a case shown in FIG. 24B, the swell of the skin S is large. Still in this state, in the present embodiment, the array chuck 40 separates from the mainspring 80 and in a state immediately before the contact with the springs 90c and 90d. In other words, a puncture action is carried out in the "idling-run interval" described above. Thus according to the present embodiment, it is possible to puncture the fine needle chip to the skin in the "idling-run interval" despite variation in swell size of the subject's skin, based on the above-described C > A + B and D > E with respect to length A to E. In other words, it is possible to puncture the skin substantially without receiving direct stress from the spring.

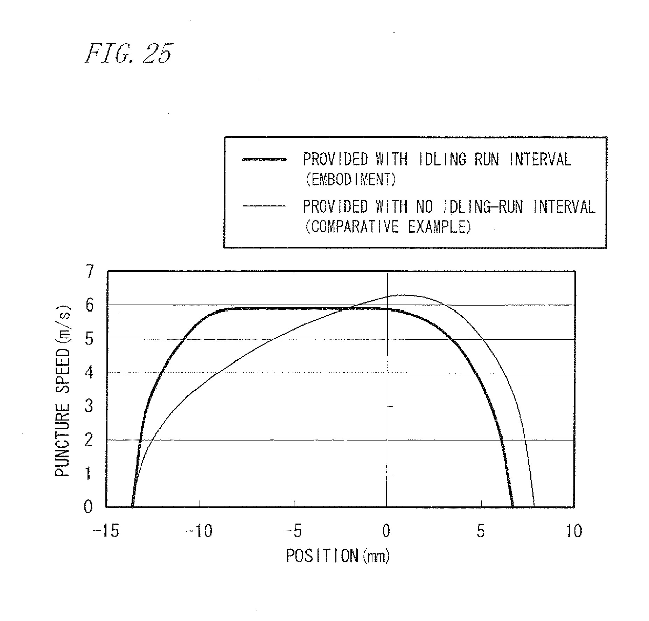

FIG. 25 is a view showing variation (influenced) in puncture speeds caused by misalignment of the puncture site due to the skin swell. In other words, FIG. 25 is a view showing an effect of the idling-run interval. In FIG. 25, a bold solid line shows a relationship between a puncture site and a puncture speed in the puncture device according to the present embodiment which is provided with the idling-run interval. A thin solid line shows a relationship between a puncture site and a puncture speed in a puncture device according to a comparative example which is not provided with the idling-run interval. In FIG. 25, point 0 (original point) on a horizontal axis represents a strike position when a skin swell is not considered. When the skin swells, the skin is punctured at a minus position on the graph. For example, when the skin swells by 5mm, it shows that the fine needle chip strikes against the skin at point "-5" (mm) on the horizontal axis. Further, in the example shown in FIG. 25, a puncture speed in the idling-run interval is set up at 6m/s. In a case where the idling-run interval is provided, it is found that when a size of the skin swell is up to 8 mm, the skin is punctured at a constant speed of 6 m/s. On the other hand, it is found that in a case where the idling-run interval is not provided, a puncture speed linearly declines in proportion with a size of the skin swell, and for example at a swell size of 5 mm, a puncture speed varies about 10% compared with a case where the idling-run interval is provided.

Specification of the drive spring, the repulsion spring, and the spacer which are used in respective puncture devices according to the above-described embodiment and comparative example is shown in Table 1. The spacer is a ring-shape member disposed on an upper side of the shaft section 51 (Refer to FIG. 22A) inserted into the drive spring in order to adjust the bias force of the drive spring.

Table-1

| | | No idling-run interval (Comparative example) | Idling-run interval (Embodiment) |
|---|---|---|---|
| Drive spring | Spring constant (N/mm) | 0.17 | 2.42 |
| | Natural length (mm) | 40 | 27.5 |
| Repulsion spring | Spring constant (N/mm) | 1.7 | 1.7 |
| | Natural length (mm) | 11 | 11 |
| Spacer (mm) | | 10 | 2.5 |

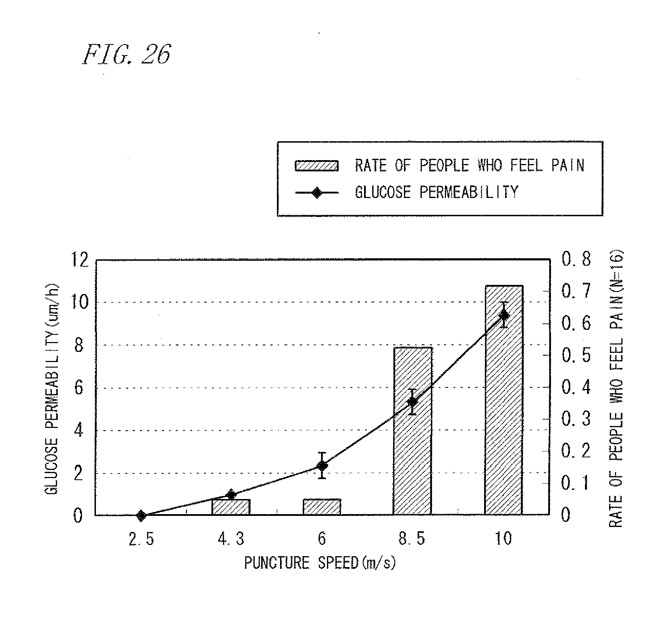

FIG. 26 is a view showing a relationship between puncture speed, glucose permeability, and a rate of people who feel pain. Basically, the higher a puncture speed is, the deeper fine pores are formed. Accordingly, a tissue fluid quantity which is extracted from the fine pores increases and glucose permeability increases. In the present embodiment, a puncture speed is a piston speed when a piston is moved by a spring. Here, provided x (m) represents compression amount, k (N/m) represents spring constant, m (kg) represents mass of piston, and v (m/s) represents puncture speed, the following relationship is established.

$$1/2 \cdot m \cdot v^2 = 1/2 \cdot k \cdot x^2 \qquad \dots \ (1)$$

A puncture speed in FIG. 26 is calculated by assigning a spring constant, a piston weight, and a compression amount in Formula (1) after appropriately adjusting units of respective numerical values. In FIG. 26, puncture is carried out under a condition where a spring constant, a piston weight, and a compression amount are adjusted and a puncture speed is 2.5 m/s, 4.3 m/s, 6 m/s, 8.5 m/s, and 10 m/s. FIG. 26 shows that glucose permeability is much influenced by a puncture speed. For example, when a puncture speed decreases from 6 m/s to 4.3 m/s, glucose permeability decreases by approximately half. As a result, it is difficult to stably measure glucose. Therefore, it is possible to form fine pores where the skin swells varied among individuals/sites are corrected, by using the puncture device which keeps a constant puncture speed, in other words, having an idling-run interval.

Further, FIG. 26 shows that when a puncture speed becomes higher than a puncture speed at approximately 8 m/s as a boundary, a rate of people who feel a pain due to puncture suddenly comes to increase. In consideration of this and a degree of glucose permeability decrease described above, it is found that 4 to 8 m/s is preferable and approximately 6 m/s is more preferable for a puncture speed.

Meanwhile the present invention is not limited to the embodiment described above and a design may be appropriately modified.

For example, forms of members configuring the piston, and methods of installing the drive spring and the repulsion spring may be appropriately modified. Further, in the embodiment described above, any ends of the drive spring and the repulsion spring are not locked to the other member but free. However, one end may be locked to the other member.

**Claims**

1. A puncture device for forming a fine pore on a skin of a subject by striking a needle against the skin, comprising:

   a piston , wherein the needle is to be attached to a distal end of the piston;
   a drive spring which has one end capable of contacting a proximal end of the piston, and moves the piston in a specific direction toward the skin; and
   a first contact section including a contact surface capable of contacting other end of the drive spring,

   wherein the one end of the drive spring and the proximal end of the piston are not fixed to each other, and/or the other end of the drive spring and the contact surface of the first contact section are not fixed to each other; and
   a length between a supposed strike position and the contact surface of the first contact section is longer than a total of a natural length of the drive spring and a length between the proximal end of the piston and a tip end of the needle, wherein the supposed strike position is where the tip end of the needle is supposed to strike against the skin.

2. The puncture device according to claim 1,
   wherein the one end of the drive spring and the proximal end of the piston are not fixed to each other, and the other end of the drive spring and the contact surface of the first contact section are not fixed to each other.

3. The puncture device according to claim 1 or 2, further comprising;

   a repulsion spring for pushing back the piston in a direction opposite to the specific direction; and
   a second contact section including a contact surface capable of contacting one end of the repulsion spring, which is on a near side to the skin,
   wherein the piston comprises a repulsion spring reception section including a contact surface capable of contacting other end of the repulsion spring, which is on a distant side from the skin,
   the one end of the repulsion spring and the contact surface of the second contact section are not fixed to each other, and/or, the other end of the repulsion spring and the contact surface of the repulsion spring reception section are not fixed to each other; and
   a length between the contact surface of the repulsion spring reception section and the tip end of the needle is longer than a total of a natural length of the repulsion spring and a length between the contact surface of the second contact section and the supposed strike position.

4. The puncture device according to claim 3,
   wherein the one end of the repulsion spring and the contact surface of the second contact section are not fixed to each other, and the other end of the repulsion spring and the contact surface of the repulsion spring reception section are not fixed to each other.

5. The puncture device according to any one of claims 1 to 4, further comprising a housing accommodating the piston, the drive spring, and the first contact section.

6. The puncture device according to claim 5,
   wherein the housing comprises a skin contact surface for contacting the skin; and
   wherein the supposed strike position is arranged inside the housing by 0.2 to 0.8 mm from the skin contact surface of the housing.

7. The puncture device according to claim 6,
   wherein the housing comprises a skin contact surface for contacting the skin; and
   wherein the supposed strike position is arranged inside the housing by 0.5 mm from the skin contact surface of the housing.

8. The puncture device according to any one of claims 1 to 7,
   wherein a puncture speed of the needle in the supposed strike position is 4 to 8 m/s.

9. The puncture device according to any one of claim 8,
   wherein the puncture speed of the needle in the supposed strike position is approximately 6 m/s.

10. The puncture device according to any one of claims 1 to 9, further comprising a spring positioning section for positioning the drive spring between the proximal end of the piston and the contact surface of the first contact section.

11. The puncture device according to claim 10,
    wherein the drive spring has tubular shape, the spring positioning section is a rod member extending from a center part of the contact surface of the first contact section through inner space of the drive spring toward the piston, and the piston comprises a passage for inserting the rod member inside the piston.

12. A fine pore formation method of forming a fine pore on a skin of a subject, comprising:

    a step of accelerating and moving a needle in a specific direction toward the skin by continuously transmitting an elastic energy which is stored in a drive spring to the needle;
    a step of releasing transmission of the elastic energy, thereafter the needle further moves to the specific direction; and
    a step of striking the needle against the skin.

**13.** The fine pore formation method according to claim 12, further comprising a step of storing the elastic energy in the drive spring by compressing the drive spring,
wherein the needle is accelerated and moved by transmitting the stored elastic energy to the needle.

**14.** The fine pore formation method according to claim 13, further comprising a step of atttaching the needle to a specific member,
wherein storing the elastic energy in the drive spring and atttaching the needle are simultaneously carried out.

**15.** The fine pore formation method according to any one of claims 12 to 14,
wherein a moving speed, when transmission of the elastic energy is released and the needle is moved further to the specific direction, is substantially constant.

**16.** The fine pore formation method according to any one of claims 12 to 15,
wherein the needle is decelerated in a case where the needle dose not strike against the skin despite movement for a specific distance in the specific direction.

FIG. 1

FIG. 2

FIG. 3

FIG. 4

FIG. 5

*FIG. 6*

*FIG. 7*

FIG. 8

*FIG. 9*

*FIG. 10*

FIG. 11

FIG. 12

*FIG. 13*

*FIG. 14*

FIG. 15

*FIG. 16*

FIG. 17A

FIG. 17B

FIG. 17C

FIG. 17D

*FIG. 18A*

140
143    143c  157a  155  156
156a
142a
141              142
142b

*FIG. 18B*

140
147  145  144
143a

*FIG. 18C*

143b    146

*FIG. 19*

*FIG. 20*

*FIG. 21*

FIG. 22A — ORDINARY STATE
FIG. 22B — TIME OF SPRING COMPRESSION (TIME OF LOAD)
FIG. 22C — TIME OF IDLING-RUN (AFTER EJECTION)
FIG. 22D — TIME OF SKIN PUNCTURE

FIG. 23A    FIG. 23B    FIG. 23C

EP 2 236 083 A1

*FIG. 24A*     *FIG. 24B*

FIG. 25

FIG. 26

*FIG. 27*

EP 2 236 083 A1

Europäisches
Patentamt

European
Patent Office

Office européen
des brevets

**EUROPEAN SEARCH REPORT**

Application Number

EP 10 15 7750

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 527 561 A (BURNS JAMES A [US]) 9 July 1985 (1985-07-09) * column 10, lines 20-65 * * figures 23-28 * | 1-10 | INV. A61B5/151 |
| X | US 2004/260326 A1 (LIPOMA MICHAEL V [US] ET AL) 23 December 2004 (2004-12-23) | 1,2,5-10 | |
| A | * paragraphs [0047] - [0050] * * figures 2-4 * | 11 | |
| X | US 4 577 630 A (NITZSCHE RAYMOND P [US] ET AL) 25 March 1986 (1986-03-25) | 1,2,5-10 | |
| A | * column 7, line 35 - column 8, line 36 * * figures 10-13 * | 11 | |
| X | US 2006/155215 A1 (CHA EUN J [KR] ET AL CHA EUN JONG [KR] ET AL) 13 July 2006 (2006-07-13) * figures 5b,6b,7b * | 1,5-10 | |
| X | WO 2008/130259 A1 (HTL STREFA SPOLKA AKCYJNA [PL]; KARBOWNICZEK JACEK [PL]; SARNA WOJCIEC) 30 October 2008 (2008-10-30) | 1,5-10 | TECHNICAL FIELDS SEARCHED (IPC) A61B |
| A | * figures 5-8 * | 11 | |

The present search report has been drawn up for all claims

| Place of search | Date of completion of the search | Examiner |
|---|---|---|
| Berlin | 20 July 2010 | Schultz, Ottmar |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 10 15 7750

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2010

| Patent document cited in search report | | Publication date | Patent family member(s) | | Publication date |
|---|---|---|---|---|---|
| US 4527561 | A | 09-07-1985 | NONE | | |
| US 2004260326 | A1 | 23-12-2004 | US | 2009105741 A1 | 23-04-2009 |
| US 4577630 | A | 25-03-1986 | CA | 1228758 A1 | 03-11-1987 |
| US 2006155215 | A1 | 13-07-2006 | WO | 2005030053 A1 | 07-04-2005 |
| | | | KR | 20050032194 A | 07-04-2005 |
| WO 2008130259 | A1 | 30-10-2008 | CA | 2680514 A1 | 30-10-2008 |
| | | | EP | 2134259 A1 | 23-12-2009 |
| | | | KR | 20100016350 A | 12-02-2010 |
| | | | US | 2010168775 A1 | 01-07-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070233011 A